# EUROPEAN PATENT APPLICATION

(11) **EP 4 834 870 A1**
(43) Date of publication of application: **04.11.2026**
(21) Application number: 24911914.0
(22) Date of filing: 07.10.2024
(51) Int. Cl.: C07C 5/333, B01J 23/887, B01J 37/04, B01J 37/08, C07B 61/00, C07C 15/46

(54) **PRODUCTION METHOD FOR ALKENYL AROMATIC COMPOUND USING DEHYDROGENATION CATALYST**

(30) Priority: 25.12.2023 JP 2023217857
(71) Applicant: Clariant Catalysts (Japan) K.K., Bunkyo-ku Tokyo 113-0021 (JP)
(72) Inventor: SAKAMOTO, Yuzuru, Toyama-shi Toyama 939-2753 (JP); KOBAYASHI,5 Masayuki, Toyama-shi Toyama 939-2753 (JP)
(74) Representative: Zusammenschluss Clariant
(86) International application number: PCT/JP2024/035818
(87) International publication number: WO 2025/142020

(57) **Abstract**

The present invention relates to a production method for an alkenyl aromatic compound using a dehydrogenation catalyst. The production method is characterized by comprising a step for bringing an alkyl aromatic compound into contact with steam inside a reactor vessel, in the presence of a dehydrogenation catalyst of the alkyl aromatic compound, wherein: the weight ratio of the steam and the alkyl aromatic compound (the ratio of water/alkyl aromatic compound) is 0.40 to 1.50, and the dehydrogenation catalyst includes 30 to 90 wt. % of iron calculated on the basis of Fe₂O₃, 1 to 50 wt. % of potassium calculated on the basis of K₂O, 1 to 50 wt. % of cerium calculated on the basis of CeO₂, and 0.1 to 15 wt. % of sodium calculated on the basis of Na₂O and does not include calcium, where the wt. % is based on the total weight of the dehydrogenation catalyst, and the weight ratio of the total amount of the K₂O and Na₂O which are alkali metal oxides and CeO₂ (the ratio of alkali metal oxides/CeO₂) is not less than 1.0.

## Description

### TECHNICAL FIELD

The present invention relates to a process for producing an alkenylaromatic compound from an alkylaromatic compound using a dehydrogenation catalyst.

### BACKGROUND OF INVENTION

The raw materials for synthetic rubbers, ABS resins and polystyrene include alkenyl aromatic compounds, typically styrene monomer. Styrene monomer is conventionally produced by dehydrogenating ethylbenzene which is an alkylaromatic compound. The dehydrogenation reaction of alkylaromatic compound is an endothermic reaction accompanied by volume expansion. For example, the dehydrogenation reaction of ethylbenzene is represented by reaction formula (1) below and is generally performed in the form of a mixture of ethylbenzene gas and water vapor (steam) under elevated temperature.

C₆H₅C₂H₅→C₆H₅C₂H₃+H₂-113 kJ/mol (1)

In the reaction, thermodynamic equilibrium can only occur at low pressures on the right side since the two molecules are produced from one molecule. Low pressure is desirable for the reaction. The reaction is endothermic which means requiring high temperature and resulting in coke formation as a side reaction. To overcome this problem water is added to the reaction. The high heat capacity of water aids in the energy transfer of the endothermic reaction and suppresses coke formation. Steam generation is expensive and, furthermore, water pressure increases the overall pressure which must be kept low to avoid adversely affecting the thermodynamic equilibrium of the reaction.

Therefore, over the last some years there has been a trend towards reducing the amount of water in the industry. The amount of water in the reaction is usually expressed as the weight ratio between the water vapor and the alkylaromatic compound (water/alkylaromatic compound ratio: W/A ratio).

Patent literature 1 (WO2017/099161) discloses an alkylaromatic compound dehydrogenation catalyst, wherein the catalyst comprises sodium (Na), calcium (Ca) and yttrium (Y) in addition to a basic catalyst comprising iron (Fe), potassium (K), cerium (Ce). The dehydrogenation catalyst shows high activity at a low W/A weight ratio of 0.5 to 1.5. Patent literature 2 (WO2022/128597) discloses likewise a catalyst showing high activity at a low W/A weight ratio, the catalyst comprises yttrium and the cross section of the dehydrogenation catalyst is toothed-wheel shape.

### CITATION LIST

### Patent Literature

Patent literature 1: WO2017/099161
Patent literature 2: WO2022/128597

### SUMMARY OF INVENTION

### Technical Problem

The conventional alkylaromatic compound dehydrogenation catalysts mentioned above have high activity even under the low W/A weight ratio condition but have a problem that the catalysts become crumbly and abraded by use which resulting in shortening the catalyst's useful lifetime.

The objective of the present invention is to provide an alkylaromatic compound dehydrogenation catalyst with suppressed abrasion at a low W/A weight ratio to be used over longer period as well as a process for producing an alkenylaromatic compound using the alkylaromatic compound dehydrogenation catalyst.

### Solution to the Problem

The inventors researched the composition of the alkylaromatic compound dehydrogenation catalyst to find that the catalyst becomes crumbly due to the addition of alkali metals, especially sodium and the abrasion rate by use of the catalyst could be suppressed by removing calcium (Ca) and controlling the amount of cerium (Ce) and alkaline metals.

The process for producing an alkenylaromatic compound of the present invention comprises a step of contacting an alkylaromatic compound with water vapor in the presence of an alkylaromatic compound dehydrogenation catalyst in a reactor, wherein the weight ratio between the water vapor and the alkylaromatic compound (water/alkylaromatic compound ratio) is 0.4 to 1.5, wherein the alkylaromatic compound dehydrogenation catalyst comprises: 30 to 90 weight percent (wt. %) of iron calculated as Fe₂O₃, 1 to 50 wt. % of potassium calculated as K₂O, 1 to 50 wt. % of cerium calculated as CeO₂, and 0.1 to 15 wt. % of sodium calculated as Na₂O, and no calcium, wherein the wt. % is based on the total weight of the dehydrogenation catalyst, and wherein the weight ratio of the total weight of the alkali metal oxides of K₂O and Na₂O to CeO₂ (alkali metal oxides/CeO₂ ratio) is 1.0 or more.

Another embodiment of the present invention is an alkylaromatic compound dehydrogenation catalyst comprising: 30 to 90 weight percent (wt. %) of iron calculated as Fe₂O₃, 1 to 50 wt. % of potassium calculated as K₂O, 1 to 50 wt. % of cerium calculated as CeO₂, and 0.1 to 15 wt. % of sodium calculated as Na₂O, and no calcium, wherein the wt. % is based on the total weight of the dehydrogenation catalyst, and wherein the weight ratio of the total weight of the alkali metal oxides of K₂O and Na₂O to CeO₂ (alkali metal oxides/CeO₂ ratio) is 1.0 or more.

Another embodiment of the present invention is a process for producing the dehydrogenation catalyst above, the process comprises steps of: (i) mixing materials and water to obtain a mixture, wherein the materials comprise an iron compound, a potassium compound, a cerium compound, a sodium compound, and no calcium; (ii) forming the mixture to obtain a formed body; and (iii) calcining the formed body.

### DESCRIPTION OF THE INVENTION

The process for producing an alkenylaromatic compound comprises a step of contacting an alkylaromatic compound with water vapor in the presence of an alkylaromatic compound dehydrogenation catalyst in a reactor.

The reactor may be either a reactor containing a single catalyst bed or a series of such reactors connected in series. The series of reactors comprises several consecutive reactors where the first reactor with the outlet connected to the inlet of the second reactor, and likewise the rest of reactors connected after.

In the process for producing an alkenylaromatic compound, the alkylaromatic compound in the feed need not all be converted to alkenylaromatic compounds, the alkylaromatic compounds in the feed could be at least partially converted to alkenylaromatic compounds. When using consecutive multiple reactors, the feed in the second reactor following the first reactor comprises the alkenyl aromatic compound produced in the first reactor and unreacted alkylaromatic compound. The feed after the second reactor comprises likewise the alkenylaromatic compound produced in the earlier reactors and unreacted alkylaromatic compound. All the alkylaromatic compound fed could be ultimately converted to an alkenylaromatic compound as the conversion of unreacted alkylaromatic compound repeatedly occur through consecutive multiple reactors in an embodiment. The alkylaromatic compound comprises ethylbenzene in an embodiment. The alkylaromatic compound is ethylbenzene in another embodiment. The alkenylaromatic compound comprises styrene in another embodiment. The alkenylaromatic compound is styrene in another embodiment.

The weight ratio between the water vapor and the alkylaromatic compound as a hydrocarbon (water/alkylaromatic compound ratio: W/A ratio) is 0.40 to 1.50, 0.40 to 1.40 in another embodiment, 0.40 to 1.30 in another embodiment, 0.40 to 1.20 in another embodiment, 0.41 to 1.40 in another embodiment, 0.42 to 1.30 in another embodiment, 0.43 to 1.25 in another embodiment, 0.44 to 1.20 in another embodiment, 0.45 to 1.15 in another embodiment, 0.46 to 1.10 in another embodiment, 0.47 to 1.10 in another embodiment, 0.48 to 1.05 in another embodiment, 0.49 to 1.00 in another embodiment, 0.50 to 0.95 in another embodiment, 0.51 to 0.85 in another embodiment, 0.52 to 0.70 in another embodiment, 0.60 to 1.40 in another embodiment, 0.65 to 1.30 in another embodiment, 0.70 to 1.23 in another embodiment, 0.75 to 1.21 in another embodiment, 0.80 to 1.20 in another embodiment, 0.82 to 1.15 in another embodiment, 0.85 to 1.15 in another embodiment, 0.87 to 1.13 in another embodiment, 0.90 to 1.11 in another embodiment, 0.95 to 1.10 in another embodiment.

The liquid hourly space velocity (LHSV) of the alkylaromatic compound is 0.1 to 5 h⁻¹ in an embodiment, 0.2 to 4.5 h⁻¹ in another embodiment, 0.3 to 4.2 h⁻¹ in another embodiment, 0.4 to 4.0 h⁻¹ in another embodiment, 0.5 to 3.5 h⁻¹ in another embodiment, 0.6 to 2.8 h⁻¹ in another embodiment, 0.7 to 1.7 h⁻¹ in another embodiment, 0.8 to 1.3 h⁻¹ in another embodiment. The LHSV refers to the ratio to the catalyst filling volume of a raw material liquid feed velocity to the continuous reactor (liquid volume flow rate at 20 °C.).

The reactor comprises an inlet for supplying the alkylaromatic compound and the water vapor and an outlet thereof in one embodiment. The temperature at the inlet (inlet temperature) is 450 to 680 °C in an embodiment, 520 to 665 °C in another embodiment, 550 to 655 °C in another embodiment, 580 to 640 °C in another embodiment, 600 to 630 °C in another embodiment.

In one embodiment, the dehydrogenation reaction of the alkylaromatic compound is an endothermic reaction as described above, and the reactor's temperature at the outlet (outlet temperature) becomes lower than the inlet temperature. The outlet temperature is 400 to 670 °C in an embodiment, 500 to 655 °C in another embodiment, 500 to 645 °C in another embodiment, 530 to 630 °C in another embodiment, 550 to 620 °C in another embodiment.

In another embodiment, the temperature inside the reactor is kept isothermal, regardless of the temperature drop caused by the reaction to increase the efficiency of the dehydrogenation reaction of the alkylaromatic compound. When the inside of the reactor is isothermally controlled, the outlet temperature of the reactor does not differ significantly from the inlet temperature. The outlet temperature is 400 to 680 °C in an embodiment, 450 to 675 °C in another embodiment, 490 to 670 °C in another embodiment, 510 to 665 °C in another embodiment, 540 to 655 °C in another embodiment, 560 to 650 °C in another embodiment, 570 to 640 °C in another embodiment, 580 to 630 °C in another embodiment, 590 to 635 °C in another embodiment, 600 to 620 °C in another embodiment.

"Inlet temperature" and "outlet temperature" refer to the temperatures measured along the direction of feed flow within about 1 cm ahead of the inlet and about 1 cm before the outlet, respectively.

The absolute pressure inside the reactor is 150 kPa or less in an embodiment,120 kPa or less in another embodiment, 110 kPa or less in another embodiment. The pressure inside the reactor is 1 kPa or less in an embodiment, 10 kPa or more in another embodiment, 30 kPa or more in another embodiment, 40 kPa or more in another embodiment, 50 kPa or more in another embodiment, 70 kPa or more in another embodiment, 90 kPa or more in another embodiment. The absolute pressure inside the reactor is 1 to 150 kPa in another embodiment, 10 to 120 kPa in another embodiment, 20 to 120 kPa in another embodiment, 25 to 110 kPa in another embodiment, 30 to 120 kPa in another embodiment, 45 to 120 kPa in another embodiment, 70 to 120 kPa in another embodiment, 70 to 110 kPa in another embodiment, 90 to 110 kPa in another embodiment.

The alkylaromatic compound dehydrogenation catalyst comprises: 30 to 90 weight percent (wt. %) of iron calculated as Fe₂O₃, 1 to 50 wt. % of potassium calculated as K₂O, 1 to 50 wt. % of cerium calculated as CeO₂, 0.1 to 15 wt. % of sodium calculated as Na₂O, and no calcium, wherein the wt. % is based on the total weight of the dehydrogenation catalyst, and wherein the weight ratio of the total weight of the alkali metal oxides of K₂O and Na₂O to CeO₂ (alkali metal oxides/CeO₂ ratio) is 1.0 or more.

The dehydrogenation catalyst comprises iron (Fe) in the form of an iron compound, wherein the iron compound is iron oxide and/or a complex oxide of iron in an embodiment. The term "complex oxide" refers to an oxide that contains two or more non-oxygen atoms in the structure of the corresponding oxide.

The iron is, based on the total weight of the dehydrogenation catalyst, calculated as Fe₂O₃, 30 to 90wt. %, 40 to 85 wt. % in another embodiment, 45 to 80 wt. % in another embodiment, 50 to 81 wt. % in another embodiment, 65 to 75 wt. % in another embodiment, 69 to 73 wt. % in another embodiment.

The dehydrogenation catalyst comprises potassium (K) in the form of a potassium compound. The potassium compound is an oxide and/or a complex oxide of potassium in an embodiment. Potassium is, based on the total weight of the dehydrogenation catalyst, calculated as K₂O, 1 to 30 wt. % in another embodiment, 8 to 26 wt. % in another embodiment, 10 to 20 wt. % in another embodiment, 11 to 18 wt. % in another embodiment, 13 to 16 wt. % in another embodiment.

The dehydrogenation catalyst comprises cerium (Ce) in the form of a cerium compound. The cerium compound is an oxide and/or a complex oxide of cerium in an embodiment. Cerium is, based on the total weight of the dehydrogenation catalyst, calculated as CeO₂, 1 to 50 wt. %, 3 to 30 wt. % in an embodiment, 5 to 26 wt. % in an embodiment, 7 to 25 wt. % in an embodiment, 7 to 24 wt. % in an embodiment, 7 to 22 wt. % in an embodiment, 8 to 20 wt. % in an embodiment, 8 to 19 wt. % in an embodiment, 9 to 18 wt. % in an embodiment, 9 to 17 wt. % in an embodiment, 10 to 16wt. % in an embodiment, 11 to 15 wt. % in an embodiment.

The dehydrogenation catalyst comprises sodium (Na) in the form of a sodium compound. The sodium compound is an oxide and/or a complex oxide of sodium in an embodiment. Sodium is, based on the total weight of the dehydrogenation catalyst, calculated as Na₂O, 0.1 to 15 wt. %, 0.5 to 13 wt. % in another embodiment, 0.7 to 12 wt. % in another embodiment, 1.0 to 11.5 wt. % in another embodiment, 1.2 to 10.0 wt. % in another embodiment, 1.4 to 9.0 wt. % in another embodiment, 1.5 to 6.0 wt. % in another embodiment, 1.7 to 5.0 wt. % in another embodiment, 1.9 to 4.0 wt. % in another embodiment, 1.5 to 3.0 wt. % in another embodiment.

In one embodiment, the weight ratio of potassium (K) to sodium (Na) in the dehydrogenation catalyst respectively calculated as K₂O and Na₂O (K₂O/Na₂O ratio) is 5 to 10, 5 to 9 in another embodiment, 5 to 8 in another embodiment, 6 to 9 in another embodiment, 6 to 8 in another embodiment, 7 to 9 in another embodiment, 7 to 8 in another embodiment.

The dehydrogenation catalyst comprises yttrium (Y) in the form of an yttrium compound in another embodiment. The yttrium compound is an oxide and/or a complex oxide of yttrium in an embodiment. Yttrium is, based on the total weight of the dehydrogenation catalyst, calculated as Y₂O₃, 0.01 to 1 wt. %. Yttrium in the catalyst is, based on the total weight of the dehydrogenation catalyst, calculated as Y₂O₃, 0.05 to 0.9 wt. % in another embodiment, 0.09 to 0.8 wt. % in another embodiment, 0.11 to 0.7 wt. % in another embodiment, 0.14 to 0.6 wt. % in another embodiment, 0.17 to 0.5 wt. % in another embodiment, 0.25 to 0.4 wt. % in another embodiment.

The dehydrogenation catalyst comprises no calcium (Ca). The term "comprises no" here means that the catalyst does not substantially comprise the component and includes embodiments that the catalyst comprises the component as an impurity. In one embodiment, calcium may be comprised in the catalyst in the amount of 0.1 wt. % or less based on the total weight of the dehydrogenation catalyst. In another embodiment, calcium may be comprised in the catalyst in the amount of 0.01 wt. % or less based on the total weight of the dehydrogenation catalyst.

The dehydrogenation catalyst has the weight ratio of the total weight of the alkali metal oxides of K₂O and Na₂O to CeO₂ (alkali metal oxides/CeO₂ weight ratio) is 1.0 or more. the weight ratio of the total weight of the alkali metal oxides of K₂O and Na₂O to CeO₂ is 1.00 to 2.50 in another embodiment, 1.01 to 2.30 in another embodiment, 1.03 to 2.10 in another embodiment, 1.04 to 1.95 in another embodiment, 1.05 to 1.98 in another embodiment, 1.06 to 1.95 in another embodiment, 1.07 to 1.90 in another embodiment, 1.08 to 1.83 in another embodiment, 1.00 to 1.50 in another embodiment, 1.01 to 1.45 in another embodiment, 1.03 to 1.40 in another embodiment, 1.04 to 1.35 in another embodiment, 1.05 to 1.30 in another embodiment, 1.06 to 1.25 in another embodiment, 1.07 to 1.20 in another embodiment, 1.08 to 1.15 in another embodiment, 1.09 to 1.79 in another embodiment, 1.10 to 1.73 in another embodiment, 1.15 to 1.70 in another embodiment, 1.19 to 1.68 in another embodiment, 1.20 to 1.66 in another embodiment, 1.23 to 1.65 in another embodiment, 1.26 to 1.62 in another embodiment, 1.29 to 1.59 in another embodiment, 1.30 to 1.57 in another embodiment, 1.31 to 1.55 in another embodiment, 1.33 to 1.54 in another embodiment, 1.36 to 1.53 in another embodiment, 1.38 to 1.51 in another embodiment, 1.40 to 1.50 in another embodiment,1.41 to 1.48 in another embodiment.

In another embodiment, the dehydrogenation catalyst may further comprise an alkali metal other than potassium and sodium. In another embodiment, the dehydrogenation catalyst comprises lithium (Li). In yet another embodiment, the alkali metal comprises in the dehydrogenation catalyst consist of potassium and sodium and does not comprise any other alkali metals.

The dehydrogenation catalyst further comprises a Group 6 element in another embodiment. The Group 6 element is selected from the group consisting of chromium (Cr), molybdenum (Mo), tungsten (W), seaborgium (Sg) and a combination thereof in one embodiment. The Group 6 element is selected from the group consisting of molybdenum (Mo), tungsten (W) and a combination thereof in another embodiment. The Group 6 element comprises molybdenum (Mo) in another embodiment. The Group 6 element is molybdenum (Mo) in another embodiment. The dehydrogenation catalyst comprises no chromium (Cr) in another embodiment.

The Group 6 element is, based on the total weight of the dehydrogenation catalyst, calculated as an oxide of Group 6 element, 0.1 to 10 wt. % in another embodiment, 0.2 to 8.0 wt. % in another embodiment, 0.3 to 6.0 wt. % in another embodiment, 0.4 to 4.0 wt. % in another embodiment, 0.5 to 2.0 wt. % in another embodiment, 0.6 to 1.0 wt. % in another embodiment.

The dehydrogenation catalyst further comprises magnesium (Mg) in another embodiment. Magnesium is, based on the total weight of the dehydrogenation catalyst, calculated as MgO, 0.3 to 10 wt. % in another embodiment, 0.5 to 8 wt. % in another embodiment, 0.6 to 3 wt. % in another embodiment, 0.7 to 1.5 wt. % in another embodiment.

The dehydrogenation catalyst optionally further comprises a noble metal in another embodiment. The noble metal is selected from the group consisting of gold, silver, platinum, palladium, rhodium, iridium, ruthenium, osmium, and a combination thereof in an embodiment. The noble metal is selected from the group consisting of gold, platinum, palladium, and a combination thereof in another embodiment. The noble metal is palladium in another embodiment.

The noble metal is present in the catalyst in an amount of from 0.1 to 200 ppm by weight, based on the total weight of the dehydrogenation catalyst. The noble metal is, based on the total weight of the dehydrogenation catalyst 1 to 150 ppm in another embodiment, 5 to 100 ppm in another embodiment, 10 to 80 ppm in another embodiment, 13 to 68 ppm in another embodiment, 14 to 42 ppm in another embodiment, 15 to 25 ppm in another embodiment.

The amount of each element, such as iron, potassium, cerium and sodium and mole ratio among the elements in the dehydrogenation catalyst can be determined by using a method known to those skilled in the art, such as elemental analysis by X-ray fluorescence analysis (XRF analysis). For example, the model ZSX Primus II manufactured by Rigaku Corporation can be used for the measurement. First, a measurement sample of the dehydrogenation catalyst is ground and then pressed at 20 MPa to prepare a test sheet approximately 3 mm thick. Subsequently, the resulting test sheet is subjected to XRF analysis. A calibration curve is separately prepared from the result of the XRF analysis for a standard substance containing an element to be measured, and quantitative calculation is performed with reference to the calibration curve. The quantity of each element thus measured can be appropriately converted to the quantity calculated as the corresponding oxide (e.g. iron is Fe₂O₃, potassium is K₂O), or converted to moles, to determine the contents and mole ratio described above.

The dehydrogenation catalyst can be a catalyst in the form of a metal oxide. The weight percent (wt. %) denotation is based on the total weight of the dehydrogenation catalyst, under the assumption that all elements are fully oxidized.

The process for producing the dehydrogenation catalyst comprises steps of: (i) mixing materials and water to obtain a mixture, wherein the materials comprise an iron compound, a potassium compound, a cerium compound, a sodium compound, and no calcium; (ii) forming the mixture to obtain a formed body; and (iii) calcining the formed body.

The iron compound as a raw material comprises iron oxide, potassium ferrite (a complex oxide of iron and potassium) or sodium ferrite (a complex oxide of iron and sodium) and a mixture thereof in an embodiment. The iron compound is iron oxide in another embodiment. Different forms of iron oxide such as red, yellow, brown and black iron oxides can be used as the iron oxide. The iron oxide as a raw material is selected from the group consisting of a red iron oxide, a yellow iron oxide, a brown iron oxide, black iron oxides and a mixture thereof in another embodiment. The iron oxide as a raw material is selected from the group consisting of a red iron oxide (hematite, Fe₂O₃), a yellow iron oxide (goethite, Fe₂O₃·H₂O) and a mixture thereof in another embodiment. The iron oxide as a raw material is a red iron oxide in another embodiment. The red iron oxide is a hematite with a corresponding crystal structure in another embodiment.

The potassium compound as a raw material is selected from the group consisting of potassium oxide, potassium hydroxide, potassium carbonate, potassium bicarbonate and a mixture thereof in an embodiment. The potassium compound as a raw material is selected from the group consisting of potassium carbonate, potassium hydroxide and a mixture thereof in another embodiment. The potassium compound as a raw material comprises potassium carbonate in another embodiment.

The cerium compound as a raw material is selected from the group consisting of cerium oxide, cerium hydroxide, cerium carbonate, cerium nitrate, and a mixture thereof in an embodiment. The cerium compound as a raw material comprises cerium carbonate in another embodiment. The cerium carbonate is selected from the group consisting of cerium carbonate hydrate, cerium hydroxy carbonate and a combination thereof in another embodiment. The cerium carbonate hydrate can comprise 40 wt. % or more of cerium calculated as CeO₂, based on the weight of cerium carbonate hydrate in another embodiment. The cerium hydroxy carbonate such as (CeCO₃OH·xH₂O), (Ce₂(CO₃)₂(OH)₂·H₂O), (Ce(CO₃)₂O·H₂O, Ce₂O(CO₃)₂·H₂O and CeO(CO₃)₂·xH₂O) are available in another embodiment.

The sodium compound as a raw material is selected from the group consisting of sodium oxide, sodium hydroxide, sodium carbonate, sodium nitrate, sodium phosphate, sodium sulfate, sodium acetate, sodium chloride, sodium sulfide and a combination thereof in an embodiment. The sodium compound comprises sodium carbonate in another embodiment.

The raw materials further comprise yttrium compound in another embodiment. The yttrium compound is selected from the group consisting of yttrium oxide, yttrium hydroxide, yttrium carbonate, yttrium nitrate, yttrium phosphate, yttrium sulfate, yttrium acetate, yttrium chloride, yttrium sulfide and a mixture thereof in an embodiment. The yttrium compound is selected from the group consisting of yttrium oxide, yttrium nitrate and a mixture thereof in another embodiment. The yttrium compound comprises yttrium oxide in another embodiment.

If the dehydrogenation catalyst comprises a Group 6 element, the raw materials comprise a compound of the Group 6 element in another embodiment. The compound of the Group 6 element as a raw material is selected from the group consisting of an oxide, a complex oxide, a salt of an oxoanion of the Group 6 element and a combination thereof in another embodiment. The raw materials comprise an oxide of the Group 6 element in another embodiment.

If the dehydrogenation catalyst comprises magnesium, the raw material is selected from the group consisting of an oxide, a complex oxide, a salt of an oxoanion of magnesium and a combination thereof in another embodiment. The raw material comprises a magnesium oxide in another embodiment.

If the dehydrogenation catalyst comprises a noble metal, the raw material comprises a noble metal compound. The noble metal compound as a raw material is selected from the group consisting of an oxide, a hydroxide, a carbonate, a nitrate, a phosphate, a sulfate, an acetate, a chloride, a sulfide of the noble metal and a combination thereof in an embodiment. The noble metal compound as a raw material comprises a nitrate of the noble metal in another embodiment.

The above-said raw materials and water are mixed to obtain a mixture. The amount of water is adjustable to be suitable for the subsequent forming or to depend on the type of raw materials. The water to mixture is 2 to 50 parts by weight against 100 parts by weight of the raw materials in an embodiment.

The mixture is formed to obtain a formed body. The mixture is formed by extrusion using matrix having desired shape of holes in an embodiment. The mixture is formed into pellets by extrusion in another embodiment. The cross section of the pellet can be selected by the matrix having holes with desired shape. The cross section of the pellet is toothed-wheel shape in an embodiment. The toothed-wheel shape may be incorporated herein by reference to WO2022/128597.

The formed body is optionally dried to remove free water in an embodiment. The drying temperature is 60 to 200 °C in another embodiment, 70 to 170 °C in another embodiment, 110 to 150 °C in another embodiment. The drying time is 5 minutes to 5 hours in an embodiment, 10 minutes to 2 hours in another embodiment, 20 minutes to 1 hour in another embodiment.

The formed body is calcined to obtain the dehydrogenation catalyst. The calcination temperature is 400 to 1300 °C in an embodiment, 500 to 1150 °C in another embodiment, 800 to 1000 °C in another embodiment. The calcination time is 3 minutes to 10 hours in an embodiment, 1 hour to 6 hours in another embodiment, 1 hour to 3 hours in another embodiment.

The process for producing the dehydrogenation catalyst can be incorporated herein by reference to WO2017/099161 and WO2022/128597 in another embodiment.

### EXAMPLE

The present invention is described by the following examples. The present invention is not limited by the examples.

The following raw materials were weighed out and mixed in a kneader while adding pure water little by little to form a paste.
- Red iron oxide (hematite crystal structure)
- Potassium carbonate
- Cerium carbonate hydrate (contains 50 wt. % of cerium calculated as CeO₂)
- Cerium carbonate hydroxide
- Sodium carbonate
- Molybdenum oxide
- Yttrium oxide
- Calcium hydroxide

The obtained paste-like mixture was shaped by extrusion in cylindrical pellets with 3 mm diameter and 5 mm long. The pellets were dried at about 130 °C for 30 minutes and then calcined at 900 °C for 2 hours. The obtained dehydrogenation catalyst composition is shown in Table 1. Table 1 exhibits the amount of each component as weight percent (wt. %) calculated as an oxide based on the total weight of the dehydrogenation catalyst. The weight ratio of potassium (K) to sodium (Na) contained in the dehydrogenation catalyst calculated as K₂O and Na₂O respectively (K₂O/Na₂O ratio), was 7.15. The total weight of the dehydrogenation catalyst was 2 kg respectively.

The dehydrogenation reaction of ethylbenzene (EB) was carried out as follows to measure the abrasion rate of the used catalyst. A reactor of 500 cc volume was filled with 100 cc of each catalyst. Water vapor and ethylbenzene (EB) were fed from an inlet to an outlet of the reactor under the following conditions. The reactor was an isothermal reactor capable of adjusting the temperature inside to keep the temperature inside as much as possible to be isothermal.
- Liquid hourly space velocity (LHSV) of ethylbenzene gas: 1.0 h⁻¹
- Water vapor/ethylbenzene weight ratio (W/A ratio): 1.0
- Absolute pressure: 51 kPa
- Reactor inlet temperature: 600 °C
- Reactor outlet temperature: 620 °C
- Reaction time: 4 days

After the reaction, the catalyst was taken out of the reactor, and the total weight of the catalyst was measured. Then a cylindrical rotary drum (inner diameter of 254 mm, height of 152 mm) equipped with one baffle plate (height of 51 mm) was charged with the catalyst and rotated at 60 rpm for 30 minutes. After the rotation was completed, the catalyst was removed from the drum and filtered through a 1 mm mesh standard sieve. The weight of the powdered catalyst filtered the sieve was measured. The percentage of the weight of the powdered catalyst relative to the total weight of the used catalyst after the reaction was calculated as the abrasion rate using the following formula. Abrasion rate (%) = powdered catalyst weight (g) ÷ catalyst weight after the reaction (g) x 100

Next, to measure the EB conversion rate, the ethylbenzene dehydrogenation reaction was carried out in the same manner as in the above-mentioned abrasion rate measurement, except for the reaction conditions shown below.
- Liquid hourly space velocity (LHSV) of ethylbenzene gas: 1.0 h⁻¹
- Water vapor/ethylbenzene weight ratio (W/A ratio): 0.68
- Absolute pressure:101 kPa
- Reaction time: 200 hours

The EB conversion was determined by the following equations after the EB concentration (weight %) in ethylbenzene gas fed into the reactor and the EB concentration (wt.%) in a product liquid that was the gas being liquefied after passed through the catalyst bed in the reactor and discharged from the outlet were measured. 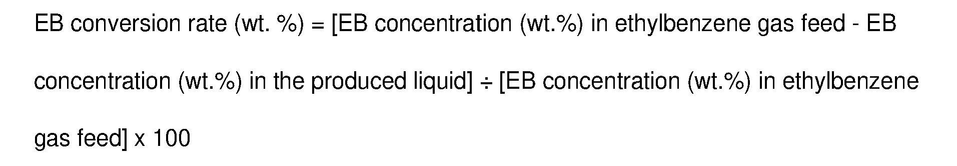 EB conversion rate (wt. %) = [EB concentration (wt.%) in ethylbenzene gas feed - EB concentration (wt.%) in the produced liquid] ÷ [EB concentration (wt.%) in ethylbenzene gas feed] x 100

The abrasion rate and the EB conversion rate of each catalyst are shown in Table 1. The abrasion rate is shown as a relative value with the value of Comparative (Com.) Example 1 set at 100. The EB conversion rate was exhibited as increase or decrease based on the standard value of Comparative Example 1

**Table 1**

| | Com. Example 1 | Example 1 |
|---|---|---|
| Fe₂O₃ | 70.7 | 71.7 |
| K₂O | 14.3 | 14.3 |
| CeO₂ | 11.0 | 11.0 |
| Na₂O | 2.0 | 2.0 |
| Y₂O₃ | 0.3 | 0.3 |
| MoO₃ | 0.7 | 0.7 |
| CaO | 1.0 | 0 |
| Abrasion rate (relative value) | 100 | 51 |
| EB conversion rate (wt%) | Standard | +2 |

### Result

The abrasion rate of the Ca-free catalyst was about half without lowering the EB conversion rate (Example 1) compared to the Ca-containing catalyst (Comparative Example 1).

Next, the weight ratio of the content of the alkali metal and cerium in the catalyst composition comprising no Ca was examined. The dehydrogenation catalysts were prepared in the same manner as Example 1 except that the obtained catalyst compositions were as shown in Table 2 and the total weight of the catalyst obtained was 10 kg. The abrasion rate was measured using the obtained dehydrogenation catalysts in the same manner as Example 1 except that the reactor used was not an isothermal reactor but a regular reactor. The inlet temperature and outlet temperature of the reactor were as follows. The result is shown in Table 2. The abrasion rate is shown as a relative value with the value of Comparative Example 2 set at 100.
- Reactor inlet temperature: 620 °C
- Reactor outlet temperature: 540 °C

**Table 2**

| | Com. Example 2 | Example 2 | Example 3 |
|---|---|---|---|
| Fe₂O₃ | 65.1 | 67.1 | 71.7 |
| K₂O | 14.3 | 14.3 | 14.3 |
| CeO₂ | 17.0 | 15.0 | 11.0 |
| Na₂O | 2.0 | 2.0 | 2.0 |
| Y₂O₃ | 0.9 | 0.9 | 0.3 |
| MoO₃ | 0.7 | 0.7 | 0.7 |
| CaO | 0 | 0 | 0 |
| (K₂O+Na₂O)/CeO₂ | 0.96 | 1.09 | 1.48 |
| Abrasion rate (relative value) | 100 | 74 | 59 |

### Result

The catalysts with the weight ratio of the total amount of alkali metal oxides K₂O and Na₂O to CeO₂ in the catalyst [(K₂O+Na₂O)/CeO₂] of 1.09 and 1.48 showed lower abrasion rates of 74 and 59 respectively (Example 2 and 3), compared to the catalyst with the weight ratio of 0.96 (Comparative Example 2).

The effects of sodium and calcium in the catalyst compositions without yttrium were investigated. The dehydrogenation catalysts were prepared, and the abrasion rate and the EB conversion rate were measured in the same manner as in Example 1, except that the obtained catalyst compositions were as shown in Table 3. The results are shown in Table 3. The abrasion rate is shown as a relative value with the value of Comparative Example 1 set at 100. The EB conversion rate was exhibited as increase or decrease based on the standard value of Comparative Example 1.

**Table 3**

| | Com. Example 3 | Com. Example 4 | Example 4 |
|---|---|---|---|
| Fe₂O₃ | 71.0 | 71.0 | 72.0 |
| K₂O | 16.3 | 14.3 | 14.3 |
| CeO₂ | 11.0 | 11.0 | 11.0 |
| Na₂O | 0 | 2.0 | 2.0 |
| Y₂O₃ | 0 | 0 | 0 |
| MoO₃ | 0.7 | 0.7 | 0.7 |
| CaO | 1.0 | 1.0 | 0 |
| Abrasion rate (relative value) | 101 | 105 | 58 |
| EB conversion rate (wt. %) | -7.1 | -2.6 | -11.2 |

### Result

The catalyst containing Na₂O showed an improvement in EB conversion rate from - 7.1% to -2.6% but the abrasion rate increased from 101 to 105 (Comparative Example 4) compared to the catalyst without Na₂O (Comparative Example 3). The catalyst containing Na₂O but not CaO had the lower abrasion rate of 58 (Example 4).

The present invention could be also provided by the embodiments below. Embodiment 1. A process for producing an alkenylaromatic compound comprising a step of contacting an alkylaromatic compound with water vapor in the presence of an alkylaromatic compound dehydrogenation catalyst in a reactor, wherein the weight ratio between the water vapor and the alkylaromatic compound (water/alkylaromatic compound ratio) is 0.40 to 1.50,
wherein the dehydrogenation catalyst comprises:
30 to 90 weight percent (wt. %) of iron calculated as Fe₂O₃,
1 to 50 wt. % of potassium calculated as K₂O,
1 to 50 wt. % of cerium calculated as CeO₂,
0.1 to 15 wt. % of sodium calculated as Na₂O, and
no calcium,
wherein the wt. % is based on the total weight of the dehydrogenation catalyst, and
wherein the weight ratio of the total weight of the alkali metal oxides of K₂O and Na₂O to CeO₂ (alkali metal oxides/CeO₂ ratio) is 1.0 or more.

Embodiment 2. The process of embodiment 1, wherein the dehydrogenation catalyst further comprises 0.01 to 1 wt. % of yttrium calculated as Y₂O₃.

Embodiment 3. The process of embodiment 1 or 2, wherein the reactor comprises an inlet for supplying the alkylaromatic compound and the water vapor and an outlet for discharging thereof, wherein the temperature at the inlet is 450 to 680 °C.

Embodiment 4. The process of any one embodiment 1 to 3, wherein the alkylaromatic compound contacts the water vapor at a pressure of 1 kPa to 150 kPa.

Embodiment 5. The process of any one of embodiment 1 to 4, wherein the alkylaromatic compound contacts the water vapor at a liquid hourly space velocity (LHSV) of 0.1 to 5 h⁻¹.

Embodiment 6. The process of any one of embodiment 1 to 5, wherein the weight ratio between the water vapor and the alkylaromatic compound (water/alkylaromatic compound ratio) is 0.40 to 1.20.

Embodiment 7. The process of any one of embodiment 1 to 6, wherein the alkylaromatic compound is ethylbenzene and the alkenylaromatic compound is styrene.

Embodiment 8. The process of any one of embodiment 1 to 7, wherein the dehydrogenation catalyst further comprises 0.1 to 10 wt. % of a Group 6 element calculated as the oxide of Group 6 element, wherein the Group 6 element is selected from the group consisting of chromium (Cr), molybdenum (Mo), tungsten (W), seaborgium (Sg) and a combination thereof.

Embodiment 9. The process of embodiment 8, wherein the Group 6 element is molybdenum (Mo).

Embodiment 10. The process of any one of embodiment 1 to 9, wherein the weight ratio of potassium (K) to sodium (Na) in the dehydrogenation catalyst respectively calculated as K₂O and Na₂O (K₂O/Na₂O ratio) is 5 to 10.

Embodiment 11. An alkylaromatic compound dehydrogenation catalyst comprises:
30 to 90 weight percent (wt. %) of iron calculated as Fe₂O₃,
1 to 50 wt. % of potassium calculated as K₂O,
1 to 50 wt. % of cerium calculated as CeO₂,
0.1 to 15 wt. % of sodium calculated as Na₂O, and
no calcium,
wherein the wt. % is based on the total weight of the dehydrogenation catalyst, and
wherein the weight ratio of the total weight of the alkali metal oxides of K₂O and Na₂O to CeO₂ (alkali metal oxides/CeO₂ ratio) is 1.0 or more.

Embodiment 12. The dehydrogenation catalyst of embodiment 11, the dehydrogenation catalyst further comprises 0.01 to 1 wt. % of yttrium calculated as Y₂O₃.

Embodiment 13. The dehydrogenation catalyst of embodiment 11, wherein the dehydrogenation catalyst further comprises 0.1 to 10 wt. % of a Group 6 element calculated as the oxide of Group 6 element, wherein the Group 6 element is selected from the group consisting of chromium (Cr), molybdenum (Mo), tungsten (W), seaborgium (Sg) and a combination thereof.

Embodiment 14. The dehydrogenation catalyst of embodiment 13, wherein the Group 6 element is molybdenum (Mo).

Embodiment 15. The dehydrogenation catalyst of embodiment 11 to 14, wherein the weight ratio of potassium (K) to sodium (Na) in the dehydrogenation catalyst respectively calculated as K₂O and Na₂O (K₂O/Na₂O ratio) is 5 to 10.

Embodiment 16. A process for producing the dehydrogenation catalyst of embodiment 11, the process comprises steps of: (i) mixing materials and water to obtain a mixture, wherein the materials comprise an iron compound, a potassium compound, a cerium compound, a sodium compound, and no calcium; (ii) forming the mixture to obtain a formed body; and (iii) calcining the formed body.

Embodiment 17. The process of embodiment 16, wherein the materials further comprise an yttrium compound.

Embodiment 18. A process for producing an alkenylaromatic compound comprising a step of:
contacting an alkylaromatic compound with water vapor in the presence of an alkylaromatic compound dehydrogenation catalyst in a reactor, wherein the weight ratio between the water vapor and the alkylaromatic compound (water/alkylaromatic compound ratio) is 0.40 to 1.50,
wherein the dehydrogenation catalyst comprises:
   30 to 90 weight percent (wt. %) of iron calculated as Fe₂O₃,
   1 to 50 wt. % of potassium calculated as K₂O,
   1 to 50 wt. % of cerium calculated as CeO₂,
   0.1 to 15 wt. % of sodium calculated as Na₂O,
   0.01 to 1 wt. % of yttrium calculated as Y₂O₃, and
   no calcium,
   wherein the wt. % is based on the total weight of the dehydrogenation catalyst, and
   wherein the weight ratio of the total weight of the alkali metal oxides of K₂O and Na₂O to CeO₂ (alkali metal oxides/CeO₂ ratio) is 1.0 or more.

Embodiment 19. An alkylaromatic compound dehydrogenation catalyst comprises:
30 to 90 weight percent (wt. %) of iron calculated as Fe₂O₃,
1 to 50 wt. % of potassium calculated as K₂O,
1 to 50 wt. % of cerium calculated as CeO₂,
0.1 to 15 wt. % of sodium calculated as Na₂O,
0.01 to 1 wt. % of yttrium calculated as Y₂O₃, and
no calcium,
wherein the wt. % is based on the total weight of the dehydrogenation catalyst, and
wherein the weight ratio of the total weight of the alkali metal oxides of K₂O and Na₂O to CeO₂ (alkali metal oxides/CeO₂ ratio) is 1.0 or more.

## Claims

1. A process for producing an alkenylaromatic compound comprising a step of contacting an alkylaromatic compound with water vapor in the presence of an alkylaromatic compound dehydrogenation catalyst in a reactor, wherein the weight ratio between the water vapor and the alkylaromatic compound (water/alkylaromatic compound ratio) is 0.40 to 1.50,
wherein the dehydrogenation catalyst comprises:
30 to 90 weight percent (wt. %) of iron calculated as Fe₂O₃,
1 to 50 wt. % of potassium calculated as K₂O,
1 to 50 wt. % of cerium calculated as CeO₂,
0.1 to 15 wt. % of sodium calculated as Na₂O, and
no calcium,
wherein the wt. % is based on the total weight of the dehydrogenation catalyst, and
wherein the weight ratio of the total weight of the alkali metal oxides of K₂O and Na₂O to CeO₂ (alkali metal oxides/CeO₂ ratio) is 1.0 or more.

2. The process of claim 1, wherein the dehydrogenation catalyst further comprises 0.01 to 1 wt. % of yttrium calculated as Y₂O₃.

3. The process of claim 1, wherein the reactor comprises an inlet for supplying the alkylaromatic compound and the water vapor and an outlet for discharging thereof, wherein the temperature at the inlet is 450 to 680 °C.

4. The process of claim 1, wherein the alkylaromatic compound contacts the water vapor at a pressure of 1 kPa to 150 kPa.

5. The process of claim 1, wherein the alkylaromatic compound contacts the water vapor at a liquid hourly space velocity (LHSV) of 0.1 to 5 h⁻¹.

6. The process of claim 1, wherein the weight ratio between the water vapor and the alkylaromatic compound (water/alkylaromatic compound ratio) is 0.40 to 1.20.

7. The process of claim 1, wherein the alkylaromatic compound is ethylbenzene and the alkenylaromatic compound is styrene.

8. The process of claim 1, wherein the dehydrogenation catalyst further comprises 0.1 to 10 wt. % of a Group 6 element calculated as the oxide of Group 6 element, wherein the Group 6 element is selected from the group consisting of chromium (Cr), molybdenum (Mo), tungsten (W), seaborgium (Sg) and a combination thereof.

9. The process of claim 8, wherein the Group 6 element is molybdenum (Mo).

10. The process of claim 1, wherein the weight ratio of potassium (K) to sodium (Na) in the dehydrogenation catalyst respectively calculated as K₂O and Na₂O (K₂O/Na₂O ratio) is 5 to 10.

11. An alkylaromatic compound dehydrogenation catalyst comprising:
30 to 90 weight percent (wt. %) of iron calculated as Fe₂O₃,
1 to 50 wt. % of potassium calculated as K₂O,
1 to 50 wt. % of cerium calculated as CeO₂,
0.1 to 15 wt. % of sodium calculated as Na₂O, and
no calcium,
wherein the wt. % is based on the total weight of the dehydrogenation catalyst, and
wherein the weight ratio of the total weight of the alkali metal oxides of K₂O and Na₂O to CeO₂ (alkali metal oxides/CeO₂ ratio) is 1.0 or more.

12. The dehydrogenation catalyst of claim 11, wherein the dehydrogenation catalyst further comprises 0.01 to 1 wt. % of yttrium calculated as Y₂O₃.

13. The dehydrogenation catalyst of claim 11, wherein the dehydrogenation catalyst further comprises 0.1 to 10 wt. % of a Group 6 element calculated as the oxide of Group 6 element, wherein the Group 6 element is selected from the group consisting of chromium (Cr), molybdenum (Mo), tungsten (W), seaborgium (Sg) and a combination thereof.

14. The dehydrogenation catalyst of claim 13, wherein the Group 6 element is molybdenum (Mo).

15. The dehydrogenation catalyst of claim 11, wherein the weight ratio of potassium (K) to sodium (Na) in the dehydrogenation catalyst respectively calculated as K₂O and Na₂O (K₂O/Na₂O ratio) is 5 to 10.

16. A process for producing the dehydrogenation catalyst of claim 11, the process comprises steps of: (i) mixing materials and water to obtain a mixture, wherein the materials comprise an iron compound, a potassium compound, a cerium compound, a sodium compound, and no calcium; (ii) forming the mixture to obtain a formed body; and (iii) calcining the formed body.

17. The process of claim 16, wherein the materials further comprise an yttrium compound.
